(19)

Europäisches
Patentamt

European
Patent Office

Office européen
des brevets

(11) **EP 4 546 348 A1**

(12) **EUROPEAN PATENT APPLICATION**

(43) Date of publication:
**30.04.2025 Bulletin 2025/18**

(21) Application number: **24208013.3**

(22) Date of filing: **22.10.2024**

(51) International Patent Classification (IPC):
*G16B 15/30* (2019.01)    *G16B 40/20* (2019.01)

(52) Cooperative Patent Classification (CPC):
**G16B 40/20; G16B 15/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.10.2023 KR 20230142144**

(71) Applicant: **LG Management Development Institute Seoul 07336 (KR)**

(72) Inventors:
- **Hwang, Doyeong**
  **07524 Seoul (KR)**
- **Kim, Youjin**
  **07796 Seoul (KR)**
- **Kim, Kiyoung**
  **07811 Seoul (KR)**
- **Yim, Soorin**
  **07801 Seoul (KR)**

(74) Representative: **BCKIP Part mbB**
**MK1**
**Landsbergerstraße 98, 3.Stock**
**80339 München (DE)**

(54) **DEVICE FOR PREDICTING PROTEIN-PROTEIN INTERACTION USING PROTEIN COMPLEX SURFACE INFORMATION BASED ON ARTIFICIAL INTELLIGENCE AND METHOD USING THE SAME**

(57)    A prediction device for predicting protein-protein interactions using protein complex surface information based on artificial intelligence includes: memory; a communicator; and a processor operably connected to the memory and the communicator, wherein the processor may be configured to: predict a structure of a protein complex based on an artificial intelligence model, extract information related to a surface of a protein complex, and provide interaction prediction data for the protein complex and an external protein based on the extracted information related to the surface of the protein complex surface.

**FIG. 8**

EP 4 546 348 A1

**Description**

**BACKGROUND**

The present disclosure generally relates to a device for predicting protein-protein interaction using protein complex surface information based on artificial intelligence and method using the same. More specifically, some embodiments of the present disclosure may predict the structure of a protein complex through an output obtained by learning a protein sequence as an input through artificial intelligence.

A new type of antigen composed of protein complexes may be derived from somatic mutations, and may be formed by the cancer cells and antigen-presenting cells of the subject. Immunogenicity refers to the activation of T cells recognizing a specific antigen. T cells may be activated by recognizing protein complexes in which major histocompatibility complex (MHC) proteins and peptide antigens are bounded. For this mechanism to occur, T cell receptors and MHC-peptide protein complexes may be physically bound to each other.

Recently, the performance of various learning models for predicting unknown structures of protein complexes has developed. However, due to noise and high cost that may occur during the learning process, it is challenging to directly utilize these models for predicting the binding of protein complexes.

Therefore, a learning method that can more accurately predict the physical structure of a protein capable of binding through the inputs of the given protein sequence may be needed.

**[Related Art Document]**

**[Patent Document]**

Korean Patent Application Publication No. 10-2022-0064958.

SUMMARY

Some embodiments of the present disclosure may extract the structure of a protein complex that may be derived from a given random protein sequence to provide immunogenicity data corresponding to that protein sequence.

According to certain embodiments of the present disclosure, a prediction device may provide accurate data on atoms constituting proteins by predicting possible 3D structures of protein complexes through iterative learning.

The problems solved by the present disclosure are not limited to those mentioned above, and other problems not mentioned may be clearly understood by those skilled in the art from the description below.

A prediction device for predicting protein-protein interactions using protein complex surface information based on artificial intelligence according to an embodiment of the present disclosure may include: a memory; a communication unit; and a processor electrically connected to the memory and the communication unit, wherein the processor may be configured to: predict the structure of a protein complex based on a first model, extract surface information of the protein complex, and provide interaction prediction data for the protein complex and an external protein based on the extracted surface information.

A method for predicting protein-protein interaction using protein complex surface information based on artificial intelligence, performed by a processor of a computer device according to an embodiment of the present disclosure, may include: a step for predicting the structure of a protein complex based on a first model; a step for extracting surface information of the protein complex; and a step for providing interaction prediction data for the protein complex and an external protein based on the extracted surface information.

In addition, other methods and systems for implementing the present disclosure and a computer-readable recording medium storing a computer program for executing the method, may further be provided.

Furthermore, a computer program stored on a medium that allows the method of implementing some embodiments of the present disclosure to be performed on a computer may further be provided.

According to certain embodiments of the present disclosure, a device for predicting protein interaction may extract the structure of a protein complex, which may be physically bound to a T cell receptor from a protein sequence, based on surface information of the protein complex.

In addition, various embodiments of the present disclosure may improve the performance of a prediction model for more precisely and efficiently searching for a binding site of a T cell receptor by using surface information of a protein complex as an input.

The effects of the present disclosure are not limited to those mentioned above, and other effects not mentioned may be clearly understood by those skilled in the art from the description below.

**BRIEF DESCRIPTION OF DRAWINGS**

FIG. 1 is a schematic block diagram of a protein interaction prediction device for predicting immunogenicity via surface information of MHC-peptide complex in accordance with various embodiments of the present disclosure.

FIG. 2 is a schematic flow diagram of a method of predicting immunogenicity using surface information of Major Histocompatibility Complex(MHC)-peptide complex in accordance with various embodiments of the present disclosure.

FIG. 3 is a flowchart of a method for obtaining MHC-peptide complex surface information according to various embodiments of the present disclosure.

FIG. 4 is diagrams showing a method of obtaining MHC-peptide complex surface information according to various embodiments of the present disclosure.

FIG. 5 is a diagram for illustrating a sampling process at an MHC-peptide complex surface in accordance with various embodiments of the present disclosure.

FIG. 6 is a diagram of a surface of an MHC-peptide complex according to various embodiments of the present disclosure.

FIG. 7 is a diagram for illustrating a convolution operation according to various embodiments of the present disclosure.

FIG. 8 is an overall flowchart of a method of predicting immunogenicity according to various embodiments of the present disclosure.

## DETAILED DESCIPTION

Throughout the present disclosure, the same reference numerals designate the same components. The present disclosure does not describe all elements of the embodiments, and general contents in the technical field of the present disclosure or duplicated content among the embodiments are omitted. The terms "unit, module, member, block" used in the specification may be implemented in software or hardware, and depending on the embodiments, multiple "units, modules, members, blocks" may be implemented as a single component, or a single "unit, module, member, block" may also include multiple components.

Throughout the specification, when a part is described as being "connected" to another part, it includes not only cases where they are directly connected but also cases where they are indirectly connected, and indirect connection includes being connected by a wireless communication network.

Furthermore, when a part is described as "comprising" a certain component, it does not mean that it excludes other components unless explicitly stated otherwise but means that it may further include other components.

Throughout the specification, when one member is described as being "on" other member, it includes not only cases where the members are in contact but also cases where another member exists between them.

Terms such as "first" and "second" are used to distinguish one component from another, and are not intended to limit the components by the aforementioned terms.

Singular expressions include plural expressions unless the context clearly indicates otherwise.

Identification codes used for each step are provided for convenience in description and do not specify the order of the steps, and each step may be carried out in a different order unless a specific order is explicitly described.

The operating principles and embodiments of the present disclosure will be described below with reference to the accompanying drawings.

The term "device according to the present disclosure" in the present disclosure encompasses various devices capable of performing operations and providing results to users. For example, the device according to the present disclosure may include a computer, server device, and portable terminal, or it may take any one of these forms.

Here, the computer may include, for example, a notebook, desktop, laptop, tablet personal computer (PC), or slate PC, equipped with a web browser.

The server device is a server that processes information by communicating with an external device, and may include an application server, a computing server, a database server, a file server, a game server, a mail server, a proxy server, and a web server.

The portable terminal is, for example, a wireless communication device ensuring portability and mobility, and may include all kinds of handheld-based wireless communication devices such as Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, smart phone, and wearable devices such as watches, rings, bracelets, anklets, necklaces, glasses, contact lenses, or head-mounted devices (HMD).

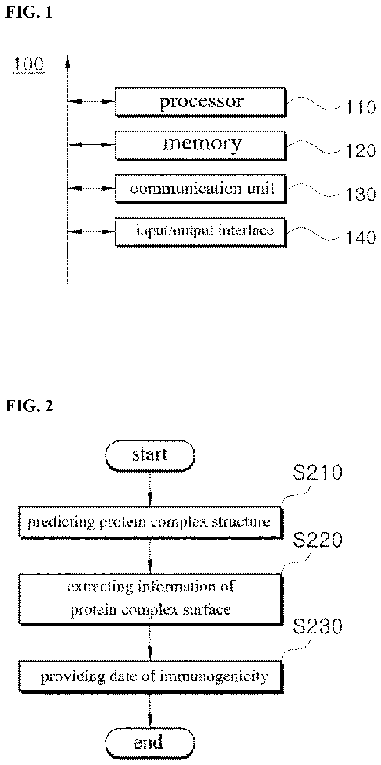

FIG. 1 is a schematic block diagram of a protein interaction prediction device for predicting immunogenicity through information related to a surface of an MHC-peptide complex according to various embodiments of the present disclosure.

A device 100 for predicting protein interaction may include one or more processors 110, memory 120, a communicator or communication unit 130, and an input and output interface 140, and the like. However, these internal components included in the device 100 for predicting protein interaction are provided for illustration purposes only, although not limited thereto. Alternatively or additionally, the device 100 for predicting protein interaction according to an embodiment of the present disclosure may perform the functions of the processor 110 through a separate processing server or a cloud server instead of a processor.

Referring to FIG. 1, the processor 110 may be configured to perform one or more operations of the device for predicting protein interaction 100 in association with the memory 120 that stores data on an algorithm for controlling the operation of components included in or associated with the device 100 for predicting protein interaction or a program that reproduces the algorithm, and the data stored in the memory 120. For example, the processor 110 and memory 120 may be implemented as separate chips. Alternatively, the processor 110 and the memory 120 may be implemented as an integrated single chip.

The processor 110 may control one or more of the components described above to implement various embodiments according to the present disclosure in the device 100 for predicting protein interaction, which are described in FIGS. 2 to 8.

The memory 120 according to an embodiment may store data performing or supporting various functions of the device for predicting protein interaction 100, programs for the operations of the processor 110, input and/or output data (e.g., images, videos, etc.), multiple application programs or applications running on the device for predicting protein interaction 100, and data or instructions for operating the device for predicting protein interaction 100. At least some of these application programs may be downloaded from an external server via wired or wireless communication.

The memory 120 may include at least one of the following types: storage medium, such as flash memory type, hard disk type, Solid State Disk (SSD) type, Silicon Disk Drive (SDD) type, multimedia card micro type, card type memory (e.g., SD or XD memory), random access memory (RAM), static random access memory (SRAM), read-only memory (ROM), electrically erasable programmable read-only memory (EEPROM), programmable read-only memory (PROM), magnetic memory, magnetic disk, and optical disk. Additionally, the memory may be a database that is separate from the device for predicting protein interaction 100, but may be connected wired or wirelessly.

The communicator or communication unit 130 according to an embodiment may include one or more components configured to communicate with external devices, including, for example, at least one of a broadcast receiving module or broadcast receiver, wired communication module or wired communicator, wireless communication module or wireless communicator, short-range communication module or short-range communicator, or location information module.

The wired communication module may include various wired communication modules such as a Local Area Network (LAN) module, a Wide Area Network (WAN) module, or a Value Added Network (VAN) module, as well as various cable communication modules such as Universal Serial Bus (USB), High Definition Multimedia Interface (HDMI), Digital Visual Interface (DVI), recommended standard 232 (RS-232), power line communication, or plain old telephone service (POTS).

The wireless communication module may include not only a WiFi module, a Wireless broadband (WiBro) module, but also a wireless communication module supporting various wireless communication methods such as Global System for Mobile Communication (GSM), Code Division Multiple Access (CDMA), Wideband Code Division Multiple Access (WCDMA), Universal Mobile Telecommunications System (UMTS), Time Division Multiple Access (TDMA), Long Term Evolution (LTE), 4G (Generation), 5G, and 6G.

The short-range communication module is for short-range communication and may support short-range communication using at least one of the following technologies: Bluetooth™, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, or Wireless Universal Serial Bus (Wireless USB).

The input and/or output interface 140 according to an embodiment serves as a channel for connecting various types of external devices to the device for predicting protein interaction 100. The input and/or output interface 140 may include, for example, but not limited to, at least one of the following: a wired and/or wireless headset port, an external charger port, a wired and/or wireless data port, a memory card port, a port for connecting a device equipped with a Subscriber Identification Module (SIM), an audio Input/Output (I/O) port, a video Input/Output (I/O) port, or an earphone port. The device for predicting protein interaction 100 may perform control related to the external device connected to the input and/or output interface 140.

At least one component may be added or omitted in accordance with the performance of the components shown in FIG. 1. It will be readily understood by those skilled in the art that the mutual positions of the components may also be modified in accordance with the performance or structure of the device.

Meanwhile, each module or component shown in FIG. 1 represents software and/or hardware components such as a Field Programmable Gate Array (FPGA) and an Application Specific Integrated Circuit (ASIC).

FIG. 2 is a schematic flow diagram of a method of predicting immunogenicity via Major Histocompatibility Complex(MHC)-peptide complex surface information in accordance with various embodiments of the present disclosure.

In step S210, a processor (e.g., the processor 110 of FIG. 1) can predict a structure of a protein complex based on a first model. For example, the first model may be a model utilized through a Docking Algorithm, and the protein complex may include an MHC-peptide complex.

In step S220, the processor can extract surface information on a surface of the protein complex. The processor can extract information about the surface of the protein complex, by focusing on at least one surface point included in a region of the surface of the protein complex, to obtain immunogenicity data from the surface of the protein complex. In step S230, the processor can provide the immunogenicity data for the protein complex based on the surface information on the surface of the protein complex.

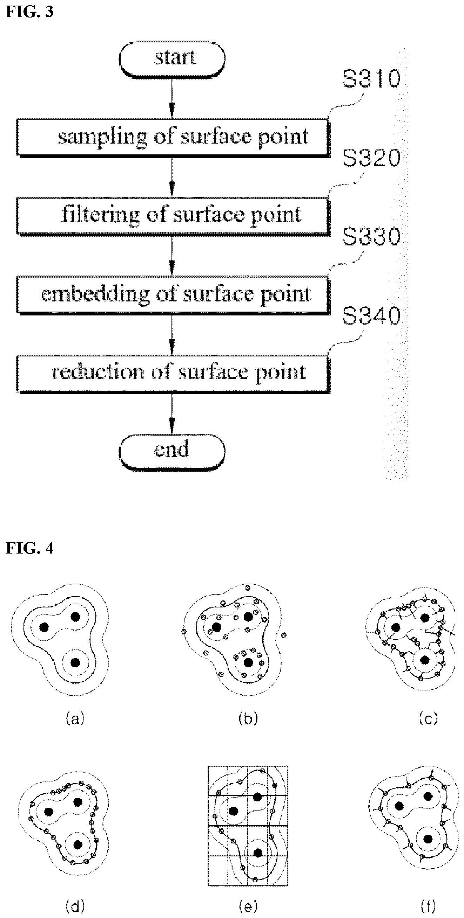

FIG. 3 is a flowchart of a method for obtaining MHC-peptide complex surface information according to various embodiments of the present disclosure.

In step S310, a processor (e.g., the processor 110 of FIG. 1) may sample at least one surface point included in a region of a surface of a protein complex. In step S310, the plurality of surface points included in a region of the surface of the protein complex may be sampled by the processor.

In step S320, the processor may filter the surface points based on peptide atoms constituting the protein complex. The processor may extract the positions of the peptide atoms based on the 3D coordinates of the peptide atoms forming the protein complex. The processor may perform the filtering of one or more surface points present or located within a predetermined distance from the position of the extracted peptide atom. The group of the filtered surface points may be referred to as a first surface point group.

In step S330, the processor can perform embedding for the filtered surface points. The processor can perform the embedding through a Convolutional Neural Network based on a geodesic distance for the first surface point group, an orientation filter for the first surface point group, and surface point features in the first surface point group.

According to some embodiments, the geodesic distance for the first surface point group may include a geodesic distance between any two different surface points in the first surface point group. The orientation filter can be computed based on the relative position and direction of any two different surface points within the first surface point group. The surface point feature may include, for example, but not limited to, six-dimensional chemical features and ten-dimensional geometric features.

In step S340, the processor can perform reduction of the surface points at a residue level based on the embedding results of step S330. At step S340, the processor can predict immunogenicity (Immunogenicity, IMM) using residue-level features to provide immunogenicity data.

According to certain embodiments, the processor can reduce surface points to the residue level. At step S340, the processor can extract residue-level features using Equation 1.

[Equation 1]

$$R_i = \frac{1}{\mathcal{N}(i)} \sum_{u \in \mathcal{N}(i)} f_u$$

According to some embodiments, the processor can reduce the number of surface points embedded at step S330 to the residue level and utilize the surface points reduced to the residue level to predict immunogenicity along with residue-level features. Here, neighboring surface points N(i) of an i-th residue-level feature Ri can be defined as surface points within a predetermined distance (e.g., r) from a nearest atom among atoms constituting Ri. The i-th residue-level feature Ri may include a mean value of the neighboring surface point features that are within the predetermined distance from the peptide atoms constituting the residue-level feature.

FIG. 4 is diagrams showing a method of obtaining MHC-peptide complex surface information according to various embodiments of the present disclosure.

FIG. 4 shows a process for sampling surface points of the protein complex. This process may involve end-to-end sampling of the protein complex surface in a rapid manner. Referring to FIG. 4, the large circles may represent peptide atoms, while the small circles may represent surface points. The contour-like lines formed by connecting the surface points can represent the surface of the MHC-peptide complex.

According to some embodiments, at FIG. 4(a), a processor (e.g., the processor 110 of FIG. 1) can sample points within a predetermined distance from each individual atom center through a smooth distance function for a peptide atom center. Subsequently, at FIG. 4(b), the processor may sample points within the predetermined distance from each individual atom center. Then, at FIG. 4(c), the processor utilizes gradient descent to find the optimal set of points representing the surface of the protein complex, and at FIG. 4(d), the processor excludes one or more points inside the folded protein. At FIG. 4(e), the processor may sample only some of the surface points based on the density of the surface points to perform point sampling of the MHC-peptide complex surface. Additionally, at FIG. 4(f), the processor can compute the gradient of the distance function at the sampled points to obtain the normal vector, which is one of the features of the surface point.

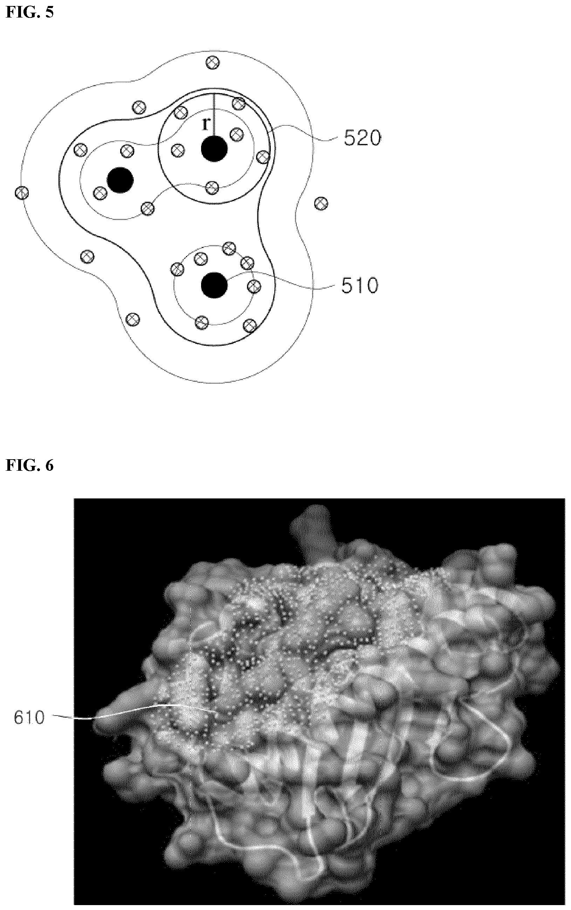

FIG. 5 is a diagram for illustrating a sampling process on a surface of an MHC-peptide complex according to various embodiments of the present disclosure. Referring to FIG. 5, a processor (e.g., the processor 110 of FIG. 1) extracts positions of peptide atoms based on the 3D coordinates of the peptide atoms constituting the MHC-peptide complex. At this point, the large circles 510 may represent peptide atoms.

According to some embodiments, the processor may filter and determine only surface points 520 that are located or exist within a predetermined distance (e.g., r in FIG. 5) from the extracted peptide atom positions as significant. Referring to FIG. 5, the processor may process or select only the surface points within the predetermined distance from one position of one peptide atom as meaningful (e.g., surface points to be sampled), and the same method can apply to other peptide atoms as well.

FIG. 6 is a diagram of a surface of an MHC-peptide complex according to various embodiments of the present disclosure. According to some embodiments of the present disclosure, a processor can iteratively filter and extract surface information from the binding site from multiple surface point groups. For example, the processor may extract the surface information of a binding site 610 shown in FIG. 6. FIG. 6 show an example of the result after filtering surface points.

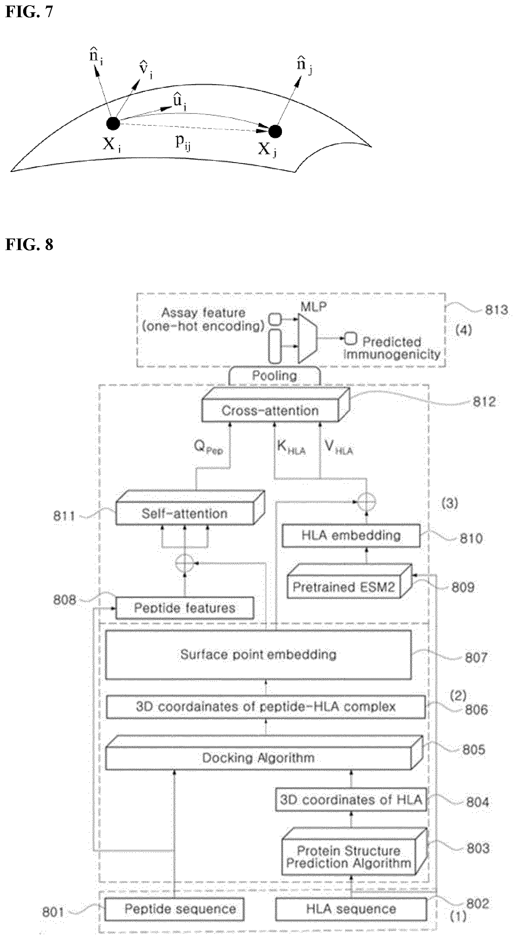

FIG. 7 is a diagram for illustrating a convolution operation according to various embodiments of the present disclosure.

Referring to FIG. 7, a processor (e.g., the processor 110 of FIG. 1) may embed surface points of a MHC-peptide complex by performing a quasi-geodesic convolution. This may be performed using Equations 2 and 3 below.

[Equation 2]

$$f_i' \leftarrow \sum_{j=1}^{N} w(d_{ij}) MLP(p_{ij}) f_j$$

In Equation 2, the processor can update surface point embedding ($f_i$) using quasi-geodesic convolution operations. Here, $w(d_{ij})$- represents a geodesic distance $w(d_{ij}) = \exp(-d_{ij}^2 / 2\sigma^2)$ between surface points i and j, with a Gaussian window $w(d_{ij}) = \exp(-d_{ij}^2 / 2\sigma^2)$ to which radius 4 is applied. $MLP(p_{ij})$ is a learnable orientation filter used for performing convolution along the MHC-peptide complex surface, calculated based on a relative position and direction of surface points i and j through position coordinates and normal vectors. $f_j$ can be represented in a total of 16 dimensions and is a 16-dimensional surface point-wise feature. This is initialized with 6-dimensional chemical features and 10-dimensional geometric features, updated accordingly.

According to certain embodiments, the processor can perform the convolution operation of FIG. 7 using Equation 3.

[Equation 3]

$$\mathbf{p}_{ij} = \underbrace{\left[ \ (\mathbf{x}_j - \mathbf{x}_i)^{\top} \ \right]}_{\text{delta of positions}} \cdot \left[ \ \hat{\mathbf{n}}_i \ \middle| \ \hat{\mathbf{u}}_i \ \middle| \ \hat{\mathbf{v}}_i \ \right]$$

$$\mathbf{q}_{ij} = \underbrace{\left[ \ (\hat{\mathbf{n}}_j - \hat{\mathbf{n}}_i)^{\top} \ \right]}_{\text{delta of normals}} \cdot \underbrace{\left[ \ \hat{\mathbf{n}}_i \ \middle| \ \hat{\mathbf{u}}_i \ \middle| \ \hat{\mathbf{v}}_i \ \right]}_{\text{local coordinate system}}$$

$$\text{Conv}(\mathbf{x}_i, \mathbf{x}_j, \mathbf{f}_j) = \underbrace{\text{Window}(d_{ij})}_{\text{quasi-geodesic patch}} \cdot \underbrace{\text{Filter}(\mathbf{p}_{ij}, \mathbf{q}_{ij})}_{\text{oriented filter}} \cdot \mathbf{f}_j$$

FIG. 8 is a flowchart illustrating a method for predicting immunogenicity according to various embodiments of the present disclosure.

Referring to FIG. 8, a peptide sequence 801 may be one of protein sequences and may be a sequence representing a subset of a protein in which amino acids are connected in a single row. For example, Human Leukocyte Antigen (HLA) and Major Histocompatibility Complex (MHC) are used interchangeably. The peptide sequence may be represented by a string indicating the order of amino acids connected by peptide bonds. HLA sequence 802 may refer to a sequence of HLA molecules corresponding to proteins that play an important role in the human immune system. Here, the HLA corresponds to a protein complex that is used to present antigens recognized mainly by immune cells such as T cells and B cells. The HLA sequence represents the amino acid sequence of HLA molecules, which may be experimentally determined using protein sequencing technology or searched through online resources.

A Protein Structure Prediction Algorithm 803 according to an embodiment may be a deep learning-based model configured or developed for predicting the 3D structure of protein. The Protein Structure Prediction Algorithm 803 may predict the 3D structure of protein from the one-dimensional amino acid sequence of a protein utilizing a technique called Markov Deep Learning. For example, the Protein Structure Prediction Algorithm 803 may include an algorithm that utilizes the ESMfold model. 3D coordinates of the HLA 804 may be 3D coordinates of the HLA predicted by the Protein Structure Prediction Algorithm 803 based on the HLA sequence 802.

A Docking Algorithm 805 according to an embodiment may be an algorithm that utilizes an operational model to predict structural changes of a protein alone or a protein-ligand complex. The Docking Algorithm 805 may predict the structural changes occurring in a ligand-bound protein structure and the effects of ligand binding. For example, the Docking Algorithm 805 may include an algorithm that utilizes a DiffDock model. The Docking Algorithm 805 may allow for structural prediction of protein complexes, protein-ligand interaction analysis, drug design, and the like.

3D coordinates of the HLA-peptide complex 806 according to an embodiment may be extracted from the 3D coordinates of the HLA-peptide complexes extracted via a second model based on the 3D coordinate of the HLA and the peptide sequence.

According to an embodiment, surface point embedding 807 may represent a feature of points for a surface of the 3D model in a vector form. The 3D model may be represented by a collection of numerous points, and the individual points may have coordinates on the 3D space. The surface point embedding 807 may perform the function of converting surface points into low-dimensional vectors to abstract and represent features of the 3D model. After performing the surface point embedding 807, the processor may reduce the feature of the 3D model to a residue level and perform self-attention 811 in combination with peptide feature 808 extracted from the peptide sequence 801.

The pre-learned ESM2 809 according to certain embodiments may be a second version of an evolutionary scale modeling (ESM) model which was previously learned. The ESM model may be a deep learning model that is utilized to understand and handle protein sequences. The ESM model may be used to predict the structure of protein complexes, inter-protein interactions, protein-ligand interactions, and the like.

Referring to FIG. 8, HLA embedding 810 may be an operation used to effectively represent HLA molecules that play an important role in the human immune system. The HLA may have unique HLA types for each individual, as each genotype has a variety of alleles. The HLA embedding 810 may be an operation that represents the HLA type as a high-dimensional vector. This operation can more easily represent similarities and differences between HLA types, with features corresponding to a specific HLA type. An embedding vector may have hundreds or more dimensions and may represent an HLA with a position on a multi-dimensional space instead of a single number.

According to an embodiment, the self-attention 811 may be one of the key components of a transformer, which is a deep learning model used in the field of natural language processing (NLP). The self-attention 811 may be a method of calculating a correlation of each position with another position for every position in a given input sequence. As a result, individual words (e.g., tokens) of the input sequence may be represented by vectors, and the self-attention 811 may calculate how much individual word vectors are associated with other word vectors. Cross attention 812 is utilized to model the interaction with other input sequences.

An array feature according to an embodiment may refer to a characteristic expressed in a multidimensional arrangement form in data. A multilayer perceptron (MLP) may be a type of artificial neural network. The MLP is a deep learning model including multiple hidden layers, and an individual hidden layer 813 may have multiple neurons.

According to some embodiments, a processor (e.g., the processor 110 in FIG. 1) may identify data related to at least one protein sequence from memory (e.g., the memory 120 in FIG. 1). For example, at least one protein sequence may comprise an HLA sequence and a peptide sequence. The processor may determine, based on the HLA sequence and the peptide sequence, whether a T cell has a structure of a protein complex that can be recognized and activated as a specific antigen.

According to certain embodiments, a processor may predict a first protein complex based on data relating to at least one protein sequence. For example, the protein complex may comprise an MHC-peptide complex. That is, the processor may provide immunogenicity data through data learned about information or a structure of whether a T cell is a protein complex that can be recognized and activated as a particular antigen.

According to some embodiments, a processor may extract 3D coordinates of HLA through a Protein Structure Prediction Algorithm based on a HLA sequence. The processor may insert the HLA sequence as an input value into the Protein Structure Prediction Algorithm, and predict a 3D coordinate (e.g., a 3D structure) of the HLA with respect to the input value. For example, the processor may extract the 3D coordinates of the HLA through a learning model based on the HLA sequence.

According to certain embodiments, a processor may extract 3D coordinates of a HLA-peptide complex through a model utilizing Docking Algorithm based on 3D coordinates and a peptide sequence of the HLA. The processor may input the 3D coordinates and the peptide sequence of the HLA as input values into the model utilizing Docking Algorithm, and predict the 3D coordinates (e.g., 3D structures) of the HLA-peptide complex with respect to the input values. That is, the processor may extract the 3D coordinates of the HLA-peptide complex through a learning model based on the peptide sequence and the 3D coordinate of the HLA extracted from the HLA sequence. By these operations, according to some embodiments, the processor can perform embedding for the surface points.

According to certain embodiment, a processor may perform self-attention by summing peptide features extracted based on a peptide sequence and positional encoding. The processor may extract an embedded HLA embedding through a pre-learned model based on the HLA sequence. Here, the pre-learned model may be, for example, but not limited to, ESM2. The processor may perform cross-attention with a result of summing the positional encoding and the HLA embedding together with a result of performing self-attention. In this case, the processor may apply individual weights to the 3D coordinates of an individual residue alpha carbon in a positional encoding operation.

Meanwhile, some embodiments of the present disclosure may be implemented in the form of a recording medium that stores instructions executable by a computer. The instructions may be stored in the form of program code and, when executed by a processor, may generate program modules to perform the operations of the disclosed embodiments. The recording medium may be implemented as a computer-readable recording medium.

The computer-readable recording medium includes any type of recording medium in which instructions that may be decrypted by a computer are stored. For example, Examples include a read only memory (ROM), a random access memory (RAM), a magnetic tape, a magnetic disk, a flash memory, an optical data storage device, and the like.

As described above, the disclosed embodiments are described with reference to the accompanying figures. A person of ordinary skill in the art may understand that the present disclosure may be implemented in a different form from the disclosed embodiments without changing the technical sprit or essential features of the present disclosure. The disclosed embodiments are illustrative and restrictive.

**[Explanation of Symbols]**

    100: protein-protein interaction prediction device
    110: processor
    120: memory
    130: communication unit
    140: input/output interface

**Claims**

1. A system comprising:

    memory; and
    a processor operably connected to the memory, the processor configured to:

        predict a structure of a protein complex based on an artificial intelligence model,
        extract information related to a surface of the protein complex, and
        provide interaction prediction data for the protein complex and an external protein based on the extracted information related to the surface of the protein complex surface.

2. The system according to claim 1, wherein:
   the protein complex comprises a major histocompatibility complex (MHC)-peptide complex.

3. The system according to claim 2, wherein:
   the processor is configured to perform sampling, filtering, embedding, reduction, and immunogenicity prediction for surface points of the protein complex to extract the information on the surface information of the protein complex.

4. The system according to claim 3, wherein:
   the processor is configured to sample the surface points of the protein complex comprised in a region of the surface of the protein complex, and to perform the filtering for one or more of the surface points that are within a predetermined distance from a peptide atom of the protein complex.

5. The system according to claim 4, wherein:
   the processor is configured to:

        extract a position of the peptide atom based on 3D coordinates of the peptide atom of the protein complex, and
        perform the filtering for one or more of the surface points that are within the predetermined distance from the extracted position of the peptide atom to identify a first surface point group.

6. The system according to claim 5, wherein:
   the processor is configured to perform the embedding through a convolutional neural network (CNN) based on a geodesic distance for the first surface point group, an orientation filter for the first surface point group, and surface point features in the first surface point group.

7. The system according to claim 6, wherein:

        the geodesic distance for the first surface point group comprises a geodesic distance between two different surface points in the first surface point group,
        the orientation filter is configured to perform calculation based on relative positions and directions of the two different surface points in the first surface point group, and
        the surface point features comprise chemical features of 6 dimensions and geometric features of 10 dimensions.

8. The system according to claim 6, wherein:
   the processor is configured to:

        perform the reduction of the surface points at residue level based on the embedding, and
        perform the immunogenicity prediction using residue-level features.

9. The system according to claim 8, wherein:
   the residue-level features are configured by calculating an average value of neighbor surface point features that are within the predetermined distance from the peptide atom.

10. A computer-implemented method comprising:

predicting a structure of a protein complex based on an artificial intelligence model; extracting information related to a surface of the protein complex; and providing interaction prediction data for the protein complex and an external protein based on the extracted information related to the surface of the protein complex surface.

11. The computer-implemented method according to claim 10, wherein:

the protein complex comprises a major histocompatibility complex(MHC)-peptide complex, and the extracting of the information related to the surface of the protein complex comprises performing sampling, filtering, embedding, reduction, and immunogenicity prediction for surface points of the protein complex.

12. The computer-implemented method according to claim 11, wherein:

the sampling comprises sampling the surface points of the protein complex comprised in a region of the surface of the protein complex, and the filtering comprises performing filtering for one or more of the surface points that are within a predetermined distance from a peptide atom of the protein complex.

13. The computer-implemented method according to claim 12, wherein:

the extracting of the information related to the surface of the protein complex comprises extracting a position of the peptide atom based on 3D coordinates of the peptide atom of the protein complex, and the filtering comprises performing filtering for one or more of the surface points that are within the predetermined distance from the extracted position of the peptide atom to identify a first surface point group.

14. The computer-implemented method for according to claim 13, the embedding comprises performing embedding through a convolutional neural network (CNN) based on a geodesic distance for the first surface point group, an orientation filter for the first surface point group, and surface point features in the first surface point group.

15. The computer-implemented method according to claim 14, wherein:

the geodesic distance for the first surface point group comprises a geodesic distance between two different surface points in the first surface point group, the orientation filter is configured to perform calculation based on relative positions and directions of the two different surface points in the first surface point group, and the surface point features comprise chemical features of 6 dimensions and geometric features of 10 dimensions.

16. A non-transitory computer-readable storage medium having instructions that, when executed by one or more processors, cause the one or more processors to:

predict a structure of a protein complex based on an artificial intelligence model; extract information related to a surface of the protein complex; and provide interaction prediction data for the protein complex and an external protein based on the extracted information related to the surface of the protein complex surface.

**FIG. 1**

100

```
┌─────────────────────────┐
│       processor         │──── 110
└─────────────────────────┘
┌─────────────────────────┐
│        memory           │──── 120
└─────────────────────────┘
┌─────────────────────────┐
│   communication unit    │──── 130
└─────────────────────────┘
┌─────────────────────────┐
│  input/output interface │──── 140
└─────────────────────────┘
```

**FIG. 2**

```
        ( start )

           │
           ▼
┌─────────────────────────────────┐
│ predicting protein complex       │──── S210
│ structure                        │
└─────────────────────────────────┘
           │
           ▼
┌─────────────────────────────────┐
│    extracting information of      │──── S220
│    protein complex surface        │
└─────────────────────────────────┘
           │
           ▼
┌─────────────────────────────────┐
│  providing date of immunogenicity │──── S230
└─────────────────────────────────┘
           │
           ▼
        ( end )
```

**FIG. 3**

```
                    ┌─────────┐
                    │  start  │
                    └────┬────┘
                         │              S310
                         ▼
        ┌────────────────────────────────┐
        │   sampling of surface point     │
        └────────────────┬───────────────┘
                         │              S320
                         ▼
        ┌────────────────────────────────┐
        │   filtering of surface point    │
        └────────────────┬───────────────┘
                         │              S330
                         ▼
        ┌────────────────────────────────┐
        │   embedding of surface point    │
        └────────────────┬───────────────┘
                         │              S340
                         ▼
        ┌────────────────────────────────┐
        │   reduction of surface point    │
        └────────────────┬───────────────┘
                         │
                         ▼
                    ┌─────────┐
                    │   end   │
                    └─────────┘
```

**FIG. 4**

(a)  (b)  (c)

(d)  (e)  (f)

**FIG. 5**

**FIG. 6**

**FIG. 7**

**FIG. 8**

Europäisches
Patentamt

European
Patent Office

Office européen
des brevets

# EUROPEAN SEARCH REPORT

**Application Number**

EP 24 20 8013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Zhang Zuobai ET AL: "A Systematic Study of Joint Representation Learning on Protein Sequences and Structures", arXiv.org, 11 March 2023 (2023-03-11), XP093255184, Retrieved from the Internet: URL:https://arxiv.org/pdf/2303.06275 [retrieved on 2025-02-28] | 1,10,16 | INV. G16B15/30 G16B40/20 |
| Y | * the whole document * * in particular * * "Methods" section * | 2-9, 11-15 | |
| X | KRIVÁK RADOSLAV ET AL: "P2Rank: machine learning based tool for rapid and accurate prediction of ligand binding sites from protein structure", JOURNAL OF CHEMINFORMATICS, [Online] vol. 10, no. 1, 1 December 2018 (2018-12-01), page 39, XP055917839, DOI: 10.1186/s13321-018-0285-8 Retrieved from the Internet: URL:https://jcheminf.biomedcentral.com/track/pdf/10.1186/s13321-018-0285-8.pdf> [retrieved on 2025-02-28] | 1,10,16 | |
| Y A | * page 5 - page 8; figure 1 * | 2,11 3-9, 12-15 | TECHNICAL FIELDS SEARCHED (IPC) G16B |

-/--

The present search report has been drawn up for all claims

| Place of search | Date of completion of the search | Examiner |
|---|---|---|
| Munich | 28 February 2025 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 20 8013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIA YING ET AL: ": A web server for ligand binding residue and pocket prediction from protein structures", PROTEIN SCIENCE, [Online] vol. 31, no. 12, 16 November 2022 (2022-11-16), XP093255165, US ISSN: 0961-8368, DOI: 10.1002/pro.4462 Retrieved from the Internet: URL:https://pmc.ncbi.nlm.nih.gov/articles/ PMC9667820/pdf/PRO-31-e4462.pdf> [retrieved on 2025-02-28] | 1,10,16 | |
| Y | * the whole document * | 2,11 | |
| A | * in particular * * section 2; figure 1 * | 3-9, 12-15 | |
| X | Sverrisson Freyr ET AL: "Fast end-to-end learning on protein surfaces", bioRxiv, 29 December 2020 (2020-12-29), XP055937055, DOI: 10.1101/2020.12.28.424589 Retrieved from the Internet: URL:https://www.biorxiv.org/content/10.110 1/2020.12.28.424589v1.full.pdf [retrieved on 2022-06-30] | 1,10,16 | **TECHNICAL FIELDS SEARCHED (IPC)** |
| Y | * the whole document * * in particular * * section 3; figures 2-5 * | 1-16 | |
| Y | US 2022/122690 A1 (LIU KAI [US] ET AL) 21 April 2022 (2022-04-21) * the whole document * * in particular * * paragraph [0009] - paragraph [0026] * | 1-16 | |

The present search report has been drawn up for all claims

| Place of search | Date of completion of the search | Examiner |
|---|---|---|
| Munich | 28 February 2025 | Rákossy, Z |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.............................................................................

& : member of the same patent family, corresponding
document

EPO FORM 1503 03.82 (P04C01)

page 2 of 2

**EP 4 546 348 A1**

## ANNEX TO THE EUROPEAN SEARCH REPORT
## ON EUROPEAN PATENT APPLICATION NO.

EP 24 20 8013

| Patent document cited in search report | Publication date | Patent family member(s) | Publication date |
|---|---|---|---|
| US 2022122690 A1 | 21-04-2022 | AU 2021308081 A1 | 17-11-2022 |
| | | BR 112023000827 A2 | 07-02-2023 |
| | | CA 3180799 A1 | 20-01-2022 |
| | | CN 115997254 A | 21-04-2023 |
| | | EP 4182924 A1 | 24-05-2023 |
| | | EP 4513509 A2 | 26-02-2025 |
| | | IL 299801 A | 01-03-2023 |
| | | JP 2023534283 A | 08-08-2023 |
| | | KR 20230042048 A | 27-03-2023 |
| | | US 2022122690 A1 | 21-04-2022 |
| | | WO 2022016125 A1 | 20-01-2022 |

**EP 4 546 348 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220064958